# EUROPEAN PATENT APPLICATION

(11) **EP 1 249 240 A1**
(43) Date of publication of application: **16.10.2002**
(21) Application number: 01900672.5
(22) Date of filing: 11.01.2001
(51) Int. Cl.: A61K 31/717, C08B 11/10, C08B 11/12, C08B 5/14, C08B 5/00

(54) **INHIBITORS AGAINST SODIUM ION ABSORPTION, AND PREVENTIVE OR THERAPEUTIC AGENTS AND FOODS CONTAINING THE SAME**

(30) Priority: 14.01.2000 JP 2000007051
(71) Applicant: OTSUKA PHARMACEUTICAL FACTORY, INC., Naruto-shi, Tokushima 772-0017 (JP)
(72) Inventor: YAMAOKA, Ippei, Tokushima-shi, Tokushima 770-0871 (JP); SAKAI, Kyota, Naruto-shi, Tokushima 779-0225 (JP); ASAGI, Kozo, Naruto-shi, Tokushima 772-0004 (JP); UESAKO, Takuzi, 51-6, Aza Minamikawamukai, Itano-gun, Tokushima 771-0220 (JP); HASHIMOTO, Kinzi, Naruto-shi, Tokushima 772-0015 (JP); KOBAYASHI, Masaru, Naruto-shi, Tokushima 772-0017 (JP); UEHARA, Tsutomu, Chiba-shi, Chiba 266-0005 (JP)
(74) Representative: HOFFMANN - EITLE
(86) International application number: JP0100095
(87) International publication number: WO01051063

(57) **Abstract**

The present invention provides a sodium ion absorption inhibitor comprising a metal salt, other than a sodium salt, of a cellulose derivatives represented by the following formula (I) as an active ingredient, whereby absorption of salt excessively present into a living body is effectively inhibited and salt excessively present is excreted outside the body positively and safely. Furthermore, the present invention provides an agent for preventing and treating diseases caused by excessive salt ingestion or diseases in which restriction on salt ingestion is required, comprising the same as an active ingredient, and foods comprising the same.

R-O-A (I)

(wherein R represents a cellulose residue and A represents a functional group having cation-exchange ability)

## Description

### TECHNICAL FIELD

The present invention relates to a sodium ion absorption inhibitor and use thereof. More particularly, the present invention relates to a sodium ion absorption inhibitor excellent in an activity of inhibiting absorption of sodium ions by excessively ingestion and the like in digestive tracts to thereby accelerate excretion of the sodium ions into feces, and its application to medicaments or foods.

### BACKGROUND OF THE INVENTION

According to the National Nutrition Survey Results published by the Ministry of Health and Welfare, Japanese have taken at least 11.5 g of salt a day since 1975, and particularly in 1993, a daily salt intake was 12.8 g. Since there is a correlation between a daily salt intake and an incidence rate of hypertension, the Ministry of Health and Welfare recommends to decrease the daily salt intake to 10 g or less so as to prevent the incidences of hypertension, stroke and the like. In the U.S., limitation of a daily salt intake is recommended similarly and the recommendation of the U.S. Joint Committee proposes that a daily salt intake of patients suffering from hypertension must be controlled to be 6 g or less.

Moreover, it is said that there is also a correlation between a salt intake and a mortality of gastric cancer. According to the data obtained so far, in a district where a salt intake is large, for example, Toyama city, Hirosaki city in Japan, and the like, the mortality of gastric cancer is high; on the other hand, in a district where a salt intake is small, for example, Beppu city, Okinawa city in Japan, and the like, the mortality of gastric cancer is low.

It is reported that dietary fibers such as alginate and the like have sodium ion adsorbing ability to some extent (*Journal of Home Economics of Japan,* vol. 39, No. 3, p.187-195 (1988)), but its adsorbing ability was still insufficient.

The excessive salt existence in the living body thus has a bad influence on the human body. Accordingly, development of new technology to effectively inhibit absorption of salt to the living body and excrete the salt excessively present is strongly desired.

Consequently, an object of the present invention is to provide a sodium ion absorption inhibitor, an agent for preventing and treating diseases caused by excessive salt ingestion or diseases in which restriction on salt ingestion is required, and a food, which can excrete excessively ingested salt outside the body positively and safely.

### DISCLOSURE OF THE INVENTION

Thus, as a result of extensive studies in order to find a component having the above activity from a broad range of natural materials, industrial materials and the like, the inventors of the present invention have found that metal salts, other than a sodium salt, of cellulose derivatives have excellent sodium ion absorption inhibiting ability, and can be applied to foods and medicaments. Thus, the present invention has been accomplished.

Accordingly, the present invention provides a sodium ion absorption inhibitor and an agent for preventing and treating diseases caused by excessive salt ingestion or diseases in which restriction on salt ingestion is required, which comprises a metal salt, other than a sodium salt, of a cellulose derivative represented by the following formula (I) as an active ingredient:

R-O-A (I)

wherein R represents a cellulose residue and A represents a functional group having cation-exchange ability.

### BRIEF DESCRIPTION OF THE DRAWINGS

Fig. 1 is a graph which shows the influence of calcium cellulose sulfate on excretion of sodium in feces and urine of normal rats (n = 5/group).

Fig. 2 is a graph which shows the influence of calcium carboxymethyl cellulose on excretion of sodium in feces and urine of normal rats (n = 5/group).

### BEST MODE FOR CARRYING OUT THE INVENTION

The cellulose derivatives represented by the above formula (I) used in the present invention are cellulose ethers wherein the hydrogen atoms of a part or all of hydroxyl groups in celluloses are replaced with a functional group A having cation-exchange ability, and the functional group A having cation-exchange ability substituted in the cellulose derivative may be one type or two or more types.

As the cellulose residue represented by R, various known celluloses may be used and the molecular weight is not particularly limited.

Examples of the above functional group A having cation-exchange ability include a carboxyl group, a sulfonic acid group, a phosphonic acid group, a phosphoric acid and the like. Preferred are groups represented by the following formulae (II) to (V):

― (alk) ― (COOH)ₙ (II)

― (alk)ₘ ― SO₃H (III)

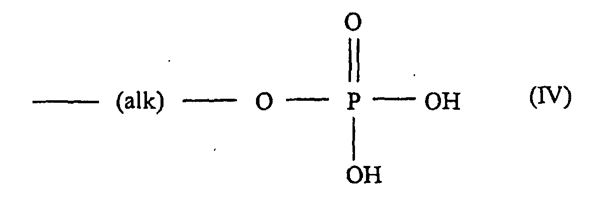

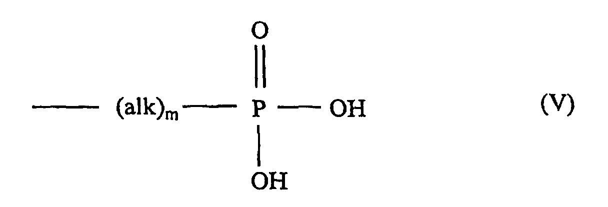

In the formulae (II) to (V), alk represents an alkylene group having 1 to 6 carbon atoms, m represents 0 or 1, and n represents an integer of 1 to 3.

Preferred examples of the functional group A are shown below.

― CH₂ ― COOH (II-1)

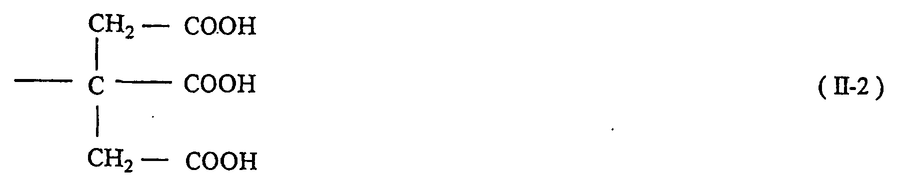

― SO₃H (III-1)

― CH₂ ― SO₃H (III-2)

― CH₂ ― CH₂ ― SO₃H (III-3)

― CH₂ ― CH₂ ― CH₂ ― SO₃H (III-4)

― CH₂ ― CH₂ ― CH₂ ― CH₂ ― SO₃H (III-5)

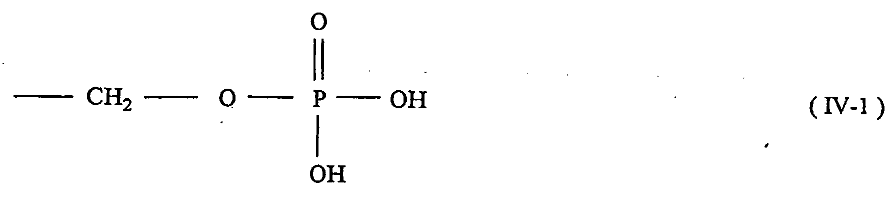

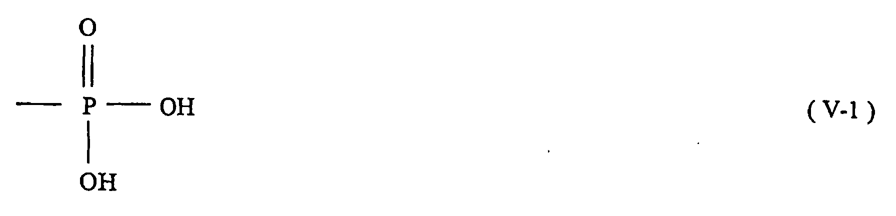

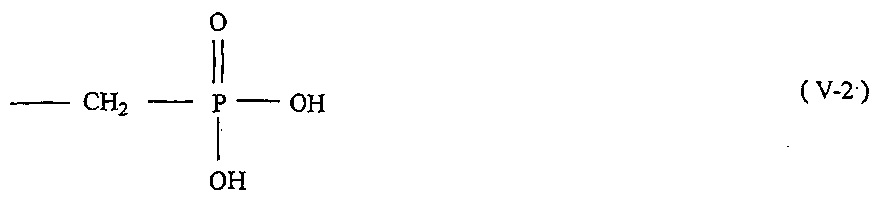

Moreover, the cellulose derivatives (I) of the present invention may have two or more kinds of the functional group A having cation-exchange ability, and are preferably cellulose derivatives comprising different functional groups having cation-exchange ability represented by the following (c-1) to (c-3) in combination.

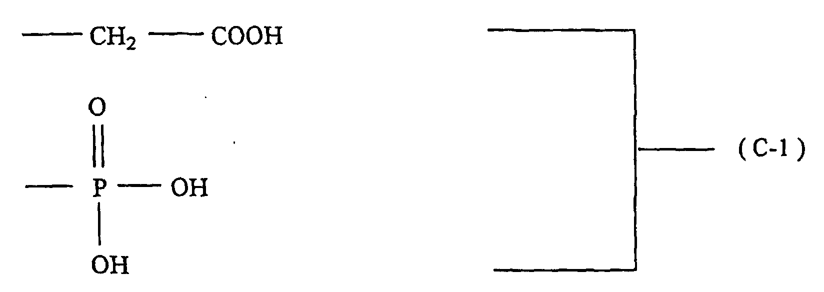

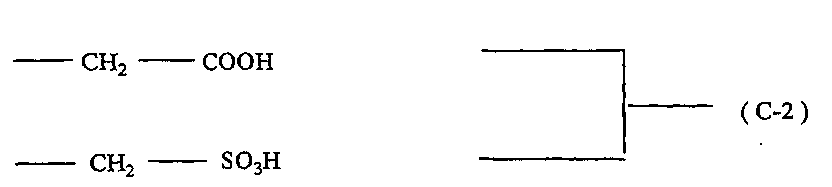

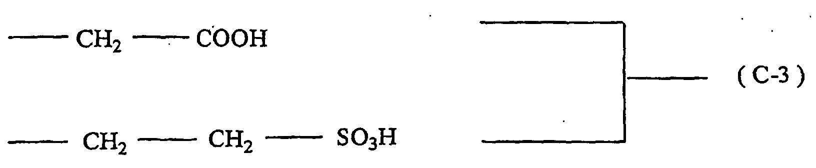

Furthermore, with regard to the group having the phosphonic acid group or phosphoric acid group represented by the above formula (IV) or (V), it is enough to exist at least one hydroxyl group in the functional group. The hydroxyl group in the phosphonic acid group or phosphoric acid group may be replaced with an alkoxy group, a phosphonic acid group, a thiol group or the like. Specifically, the functional group A having cation-exchange ability also includes the following groups:

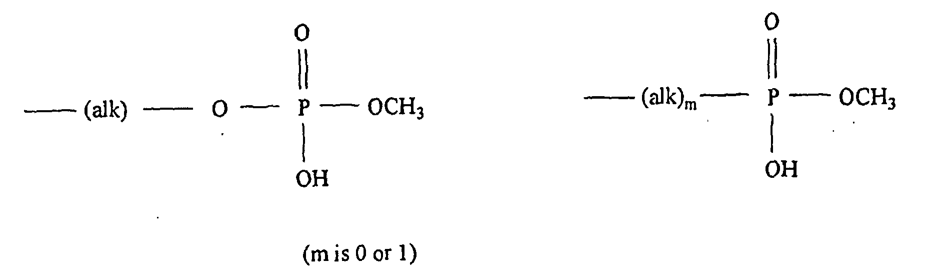

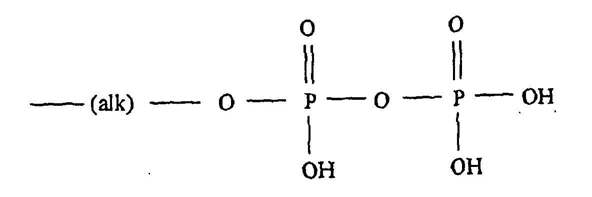

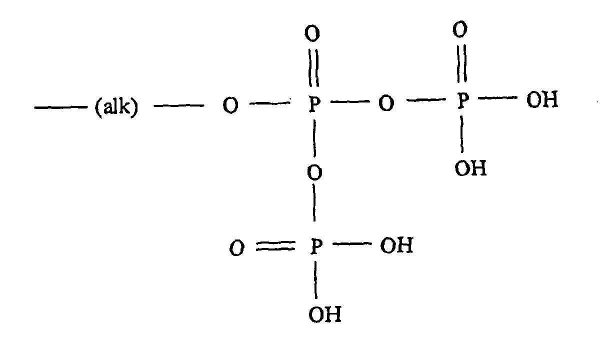

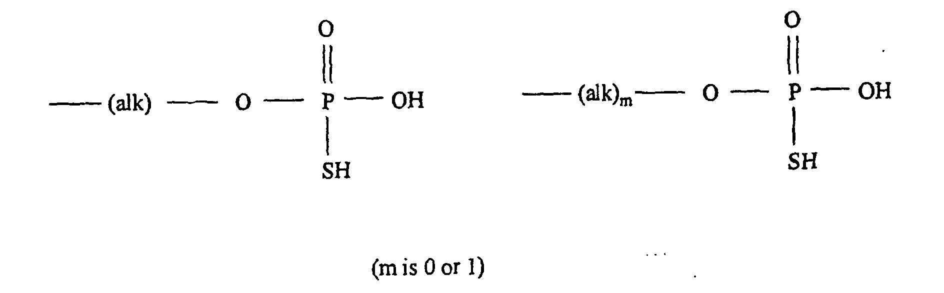

The cellulose derivatives represented by the formula (I) for use in the present invention can be produced industrially or are commercially available.

The active ingredient of the present invention is a metal salt, other than a sodium salt, of the cellulose derivatives represented by the above formula (I). Each of the cellulose derivatives represented by the above formula (I) has a functional group having cation-exchange ability, such as a carboxyl group, a sulfonic acid group or the like, and most of the functional groups are sodium salts. Therefore, the metal salt of the present invention may be formed by replacing sodium with other metal, such as calcium or the like. Examples of the metal usable for the replacement include all metals, such as alkali metal salts, other than a sodium salt, including potassium, *etc.,* alkaline earth metals including calcium, magnesium, *etc.,* iron and the like. Among these, potassium, calcium, magnesium and iron are particularly preferred.

The resulting metal salt of the cellulose derivatives may be used directly or optionally after further purification by alcohol precipitation, ion exchange resin chromatography, gel filtration chromatography or the like.

The metal salt of the cellulose derivatives of the present invention has sodium ion absorption inhibiting ability, and, particularly when it is orally administered, it inhibits absorption of sodium ions in digestive tracts and accelerates their excretion.

Consequently, the metal salt of the cellulose derivatives of the present invention is effective as a sodium ion absorption inhibitor, and can be contained in medicaments, various foods and the like. Particularly, it can alleviate salt restriction for patients suffering from various diseases caused by excessive salt ingestion, such as hypertension, gastric cancer, stroke, renal failure and osteoporosis, so that it is useful as medicaments for preventing and treating such diseases. Also, since it excretes a large amount of sodium ions into feces, it is particularly useful for excreting sodium ions from patients exhibiting reduced sodium excretion from the kidney, such as renal failure and the like.

The sodium ion absorption inhibitor and medicament (preventive and therapeutic agent) comprising the metal salt of the cellulose derivatives of the present invention can be produced by processing the metal salt of the cellulose derivatives obtained by the above method into various forms according to usual methods. Examples include solid form products, liquid form products, emulsion form products, paste form products and the like.

The sodium ion absorption inhibitor of the present invention can be applied to foods effectively. Examples of the foods containing the sodium ion absorption inhibitor include any one of those which can be ingested immediately as such, are ingested after cooking and the like, and are premixed materials for use in food production. The solid forms may be any of powder forms, granular forms and solid forms, and examples include various confectionery, such as biscuits, cookies, cakes, snacks, rice crackers and the like, bread and powder drinks (powder coffee, cocoa and the like). Examples of the liquid form, emulsion form and paste form products include various drinks, such as juices, carbonated drinks, lactic acid drinks and the like.

Examples of the medicament of the present invention include tablets, powders, granules, fine particles, solutions and the like, and these preparations can be produced by formulating the metal salt of the cellulose derivatives of the present invention together with a pharmaceutically acceptable carrier according to usual methods.

The metal salt of the cellulose derivatives of the present invention has ability to excrete and absorb about 1 g of salt (about 400 mg as sodium ions) based on about 10 g of the metal salt. Thus, using this ability as a standard, it is preferred to take it in an amount of about 0.5 to 50 g per day calculated as the above metal salt of the cellulose derivatives.

### Examples

The present invention will be explained in further detail with reference to Examples, but the present invention is not restricted thereto.

### Example 1

Effect of calcium cellulose sulfate on inhibition of sodium absorption was compared and examined using normal rats.

### (1) Production of calcium cellulose sulfate

Into a 5 liter-volume beaker containing 1 liter of distilled water was added 50 g of sodium salt of cellulose sulfate (manufactured by Acros, U.S.), which was completely dissolved under stirring. Then, 300 ml of an aqueous solution of 93 g of calcium chloride dihydrate (manufactured by Katayama Chemical) was added thereto, followed by stirring for 30 minutes. One liter of isopropyl alcohol was added thereto, followed by stirring for further 1 hour. Then, the mixture was allowed to stand overnight. The supernatant was removed by decantation and the residue was centrifuged to obtain a precipitate. The precipitate was again suspended into 1 liter of isopropyl alcohol, and the mixture was stirred for 30 minutes and then allowed to stand overnight. The supernatant was removed by decantation and the residue was centrifuged to obtain a precipitate. The precipitate was dried under reduced pressure to obtain 44 g of calcium cellulose sulfate as a white powder.

### (2) Materials and Method

Nine-weeks-old Wistar male rats (Charles River Japan, Inc.) were used as the experimental animal. After one night starvation, the rats were transferred into an individual metabolism cage and accustomed to a powder feed under a restricted feeding (20 g/rat/day) using a cellulose feed (casein 20% (w/w, the same in the following), α-corn starch 69.5%, soybean oil 5%, sucrose 5%, AIN-76 vitamin mix 1%, AIN-76 mineral mix 3.5%, salt 1% and cellulose 5%) for 3 days. Thereafter, the body weights of the rats were measured, and they were divided into groups (n = 5/group) based on the amounts of feed intake and body weights during the accustoming period. Namely, there were set a cellulose group wherein the cellulose feed was ingested, a 2%, calcium cellulose sulfate group wherein a feed obtained by replacing 40% of the cellulose in the cellulose feed with calcium cellulose sulfate was ingested and a 5% calcium cellulose sulfate group wherein a feed obtained by replacing all the cellulose in the cellulose feed with calcium cellulose sulfate was ingested.

After one night starvation, the rats of each group were reared with each test feed under a restricted feeding (20 g/rat/day) for 2 days. On the 2nd day of the test feed rearing, feces and urine were collected for 24 hours from 7 pm. By the way, during the test period, the rats of each group were allowed to ingest distilled water freely. The feces ingested were dried at 70°C for 4 days and the dry weights were determined. Thereafter, the feces were incinerated (500°C or higher, 36 hours) and sodium was measured by an atomic absorption analysis. The urine collected was subjected to a measurement of sodium in urine by an ion electrode analysis based on the weight difference of the urine-collecting cup, the specific gravity being assumed as 1.0.

### (3) Results

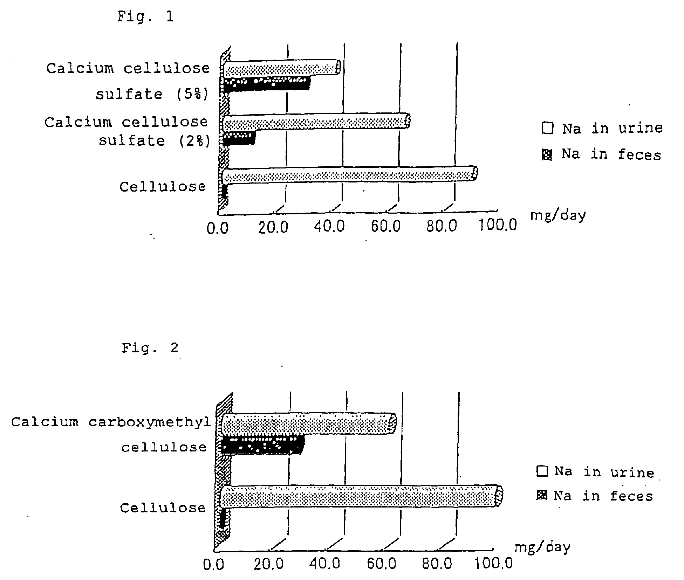

Fig. 1 shows excretion of sodium in urine and excretion of sodium in feces.

In the cellulose group, 89.6±6.5 mg/day of excretion of sodium in urine was observed, while 65.3±4.5 mg/day was observed in the 2% calcium cellulose sulfate group and 40.3±3.4 mg/day in the 5% calcium cellulose sulfate group. Thus, dose-dependent decrease of excretion of sodium in urine by the ingestion of calcium cellulose sulfate was confirmed.

On the other hand, regarding excretion of sodium in feces, 10.4±2.2 mg/day was observed in the 2% calcium cellulose sulfate group and 29.9±7.5 mg/day in the 5% calcium cellulose sulfate as compared with 0.2±0.1 mg/day in the cellulose group. Thus, dose-dependent increase of excretion of sodium in feces by the ingestion of calcium cellulose sulfate was confirmed.

### (3) Summary

From these results, it was shown that calcium cellulose sulfate has an activity of excreting ingested sodium from feces and inhibiting absorption of sodium into the living body.

### Example 2

### (1) Production of calcium cellulose sulfate

Into a 5 liter-volume beaker containing 5 liters of distilled water was added 20 g of sodium salt of cellulose sulfate (manufactured by Across, U.S.), which was completely dissolved under stirring. Then, 200 ml of an aqueous solution of 100 g of calcium chloride dihydrate (manufactured by Katayama Chemical) was added thereto, followed by stirring for 2 hours. The formed precipitate was collected by filtration, which was then placed in a dialysis tube made of cellulose (Spectra/Por (registered trademark) 1, MWCO6000-8000) and subjected to dialysis to remove low-molecular-weight substances and salts. The product was dried under reduced pressure to obtain 20 g of calcium cellulose sulfate.
(2) With regard to the calcium cellulose sulfate obtained in the above, effect on inhibition of sodium absorption was examined in a similar manner to Example 1 to obtain almost equal results as in Example 1.

### Example 3

Effect of calcium carboxymethyl cellulose on inhibition of sodium absorption was compared and examined using normal rats.

### (1) Materials and Method

Six-weeks-old Wistar male rats(Charles River Japan, Inc.) were used as the experimental animal. After one night starvation, the rats were divided into groups based on the body weights and then transferred into an individual metabolism cage, and the rats were allowed to ingest each test feed freely for six days. There were set a cellulose group wherein a cellulose feed (casein 20% (w/w, the same in the following), α-corn starch 69.5%, soybean oil 5%, sucrose 5%, AIN-76 vitamin mix 1%, AIN-76 mineral mix 3.5%, salt 1% and cellulose 5%) was ingested and a calcium carboxymethyl cellulose group wherein a feed obtained by replacing all the cellulose in the cellulose feed with calcium carboxymethyl cellulose was ingested.

On every 4th to 6th day of the test feed rearing, urines and feces were collected for 24 hours from 7 pm for 3 days. By the way, during the test period, the rats of each group were allowed to ingest distilled water freely. The feces ingested were dried at 70°C for 4 days and the dry weights were determined. Thereafter, the feces were incinerated (500°C or higher, 36 hours) and sodium was measured by an atomic absorption analysis. The urine collected was subjected to a measurement of sodium in urine by an ion electrode analysis based on the weight difference of the urine-collecting cup, the specific gravity being assumed as 1.0.

### (2) Results

Fig. 2 shows excretion of sodium in urine and excretion of sodium in feces for each group.

In the cellulose group, 98.4±4.2 mg/day of excretion of sodium in urine was observed, while 60.0±3.1 mg/day was observed in the calcium carboxymethyl cellulose group. Thus, decrease of excretion of sodium in urine by the ingestion of calcium carboxymethyl cellulose was confirmed. By the way, there was no difference in amount of ingested sodium in both groups.

On the other hand, regarding excretion of sodium in feces, 27.7±11.5 mg/day was observed in the calcium carboxymethyl cellulose group as compared with 0.1±0.0 mg/day in the cellulose group. Thus, increase of excretion of sodium in feces by the ingestion of calcium carboxymethyl cellulose was confirmed.

### (3) Summary

From these results, it was shown that calcium carboxymethyl cellulose has an activity of excreting ingested sodium from feces and inhibiting absorption of sodium into a living body.

### Example 4

Using each calcium salt of a cellulose derivative having the group of the above formula (III-2) (Cas RN = 9015-17-2; which means the registry number in Chemical Abstracts, the same in the following), a cellulose derivative having the group of the above formula (III-4) (Cas RN = 39322-23-1) or a cellulose derivative having the group of the above formula (III-5) (Cas RN = 39346-59-3) as the functional group A, the performance was evaluated in a similar manner to Example 1, whereby similar results of inhibiting sodium absorption to those in Example 1 were obtained in all the cases.

### Example 5

Using each calcium salt of a cellulose derivative having the group of the above formula (II-2) (Cas RN = 9032-38-6), a cellulose derivative having the group of the above formula (IV-1) (Cas RN = 9015-15-0), a cellulose derivative having the group of the above formula (V-1) (Cas RN = 9015-14-9) or a cellulose derivative having the group of the above formula (V-2) (trade name PPM-cellulose, manufactured by Broun) as the functional group A, the performance was evaluated in a similar manner to Example 3, whereby similar results of inhibiting sodium absorption to those in Example 3 were obtained in all the cases.

### Example 6

Using each calcium salt of a cellulose derivative having the two groups composed of the combination of the above (C-2) (Cas RN = 57285-70-8) or a cellulose derivative having the two groups composed of the combination of the above (C-3) (Cas RN = 39454-65-4) as the functional group A, the performance was evaluated in a similar manner to Example 1, whereby similar results of inhibiting sodium absorption to those in Example 1 were obtained in all the cases.

### Example 7

Using a calcium salt of a cellulose derivative having the two groups composed of the combination of the above (C-1) (Cas RN = 106008-68-8) as the functional group A, the performance was evaluated in a similar manner to Example 3, whereby similar results of inhibiting sodium absorption to those in Example 3 were obtained.

### Example 8

### (Production of fine particles)

A mixture composed of 70 parts by weight of the calcium cellulose sulfate used in Example 1 or the calcium carboxymethyl cellulose used in Example 3, 20 parts by weight of lactose and 10 parts by weight of corn starch was subjected to fluidized bed granulation with a 5% aqueous solution of hydroxypropylmethyl cellulose to obtain fine particles.

### Example 9

### (Production of biscuits)

In the following example, the term "part(s)" means part(s) by weight.

Eight parts of shortening and 18 parts of sugar are mixed, and 42 parts of soft wheat flour, 7.5 parts of the calcium cellulose sulfate used in Example 1, 0.8 part of baking powder, 16 parts of egg, 1 part of glucose and 25 parts of water were added thereto, followed by stirring to prepare dough. This dough is rolled to a sheet of 5 mm in thickness, and pieces are stamped out from the sheet, each piece having 16 to 17 g in weight, and then baked in an oven of 90°C for 32 to 36 minutes. As a result, about 12 g per piece of biscuits was obtained. It is calculated that 12 g of the biscuit contains 1 g of the calcium cellulose sulfate. That is, about 40 mg of sodium ions (about 100 mg as salt) can be adsorbed by eating one piece of the biscuits.

### Example 10

### (Production of a drink)

With ion exchange water, 12.5 g of granulated sugar, 0.2 g of citric acid crystals and 1 g of the calcium cellulose sulfate used in Example 1 were filled up to 100 ml, and the resulting mixture was bottled and then sterilized at 80°C for 10 minutes to obtain a drink containing the calcium cellulose sulfate.

### INDUSTRIAL APPLICABILITY

The metal salt of the cellulose derivatives of the present invention inhibits absorption of sodium ions in the digestive tract, reduces their absorption rate into the body, and particularly has excellent activity in excreting sodium ions into feces. Accordingly, it can be included in various foods and the like as an excellent sodium ion absorption inhibitor or a sodium ion excretion accelerator by incorporating an effective dosage.

Also, excellent agents for preventing and treating diseases caused by excessive salt ingestion or diseases in which restriction on salt ingestion is required can be obtained by including an effective amount of the metal salt of the cellulose derivatives. Particularly, since the amount of excreted sodium in feces is large, the metal salt of the cellulose derivatives of the present invention is extremely useful for patients exhibiting reduced sodium excretion from the kidney, such as renal failure and the like.

## Claims

1. A sodium ion absorption inhibitor, comprising a metal salt, other than a sodium salt, of a cellulose derivative represented by the following formula (I) as an active ingredient:
R-O-A (I)
wherein in the formula (I), R represents a cellulose residue and A represents a functional group having cation-exchange ability.

2. The sodium ion absorption inhibitor according to claim 1, wherein the functional group having cation-exchange ability is at least one selected from the groups represented by the following formulae (II) to (V):
― (alk) ― (COOH)ₙ (II)
― (alk)ₘ― SO₃H (III) 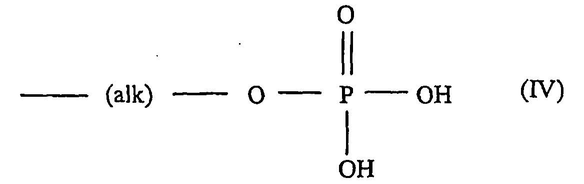 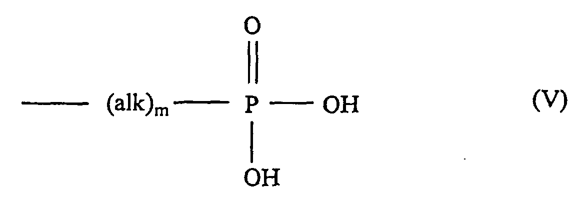
wherein in the formulae (II) to (V), alk represents an alkylene group having 1 to 6 carbon atoms, m represents 0 or 1, and n represents an integer of 1 to 3.

3. The sodium ion absorption inhibitor according to claim 2, wherein the functional group having cation-exchange ability is at least one selected from the following formulae (II-1), (II-2), (III-1) to (III-5), (IV-1), (V-1), and (V-2):
― CH₂ ― COOH (II-1) 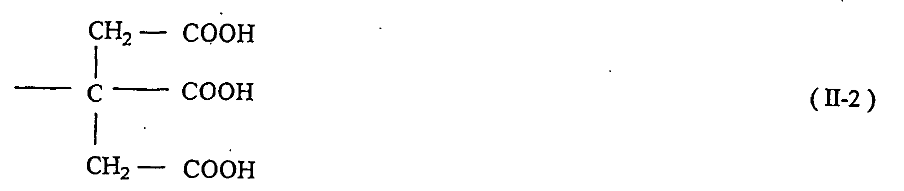
― SO₃H (III-1)
― CH₂ ― SO₃H (III-2)
― CH₂ ― CH₂ ― SO₃H (III-3)
― CH₂ ― CH₂ ― CH₂ ― SO₃H (III-4)
― CH₂ ― CH₂ ― CH₂ ― CH₂ ― SO₃H (III-5)

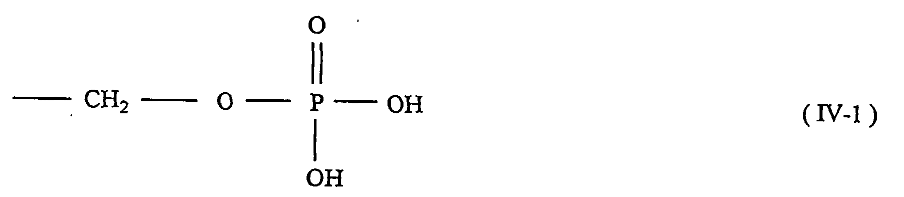

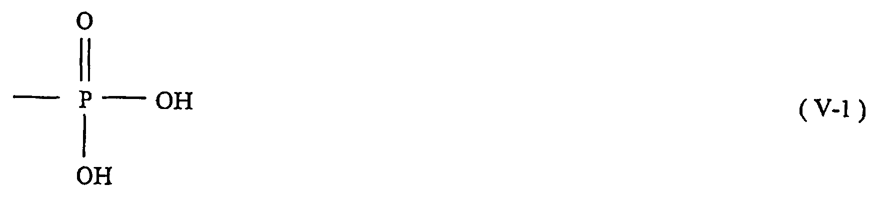

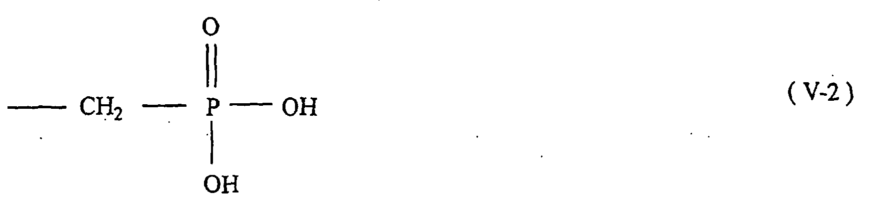

4. The sodium ion absorption inhibitor according to any one of claims 1 to 3, wherein the cellulose derivative comprises different functional groups having cation-exchange ability.

5. The sodium ion absorption inhibitor according to claim 4, wherein the different functional groups having cation-exchange ability are a combination selected from the following (c-1) to (c-3):

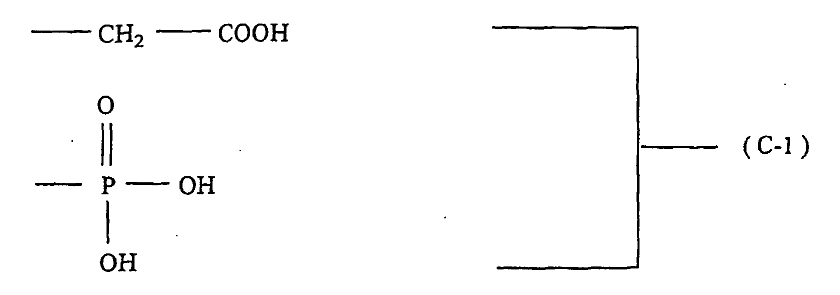

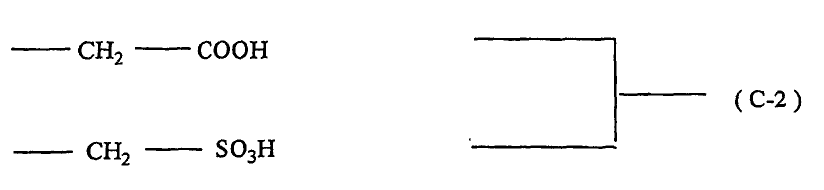

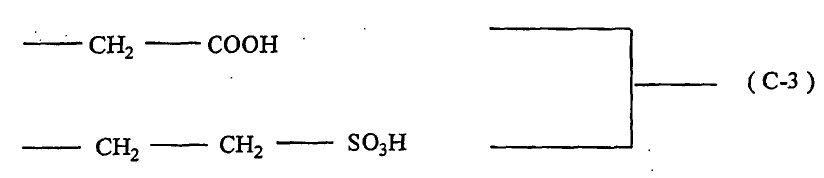

6. An agent for preventing and treating a disease caused by excessive salt ingestion or a disease restriction on salt ingestion is required, which comprises the metal salt, other than a sodium salt, of the cellulose derivative according to any one of claims 1 to 5 as an active ingredient.

7. A food comprising the metal salt, other than a sodium salt, of the cellulose derivative according to any one of claims 1 to 5.
